(19) Europäisches Patentamt
European Patent Office
Office européen des brevets

(11) Publication number: **0 230 125 A1**

(12) # EUROPEAN PATENT APPLICATION

(21) Application number: 86309720.0

(51) Int. Cl.[4]: **A61K 31/615**

(22) Date of filing: 12.12.86

Claims for the following Contracting States: AT + ES + GR.

(30) Priority: 23.12.85 US 812568

(43) Date of publication of application: 29.07.87 Bulletin 87/31

(84) Designated Contracting States: AT BE CH DE ES FR GB GR IT LI LU NL SE

(71) Applicant: **MERCK & CO. INC.**
126, East Lincoln Avenue P.O. Box 2000
Rahway New Jersey 07065(US)

(72) Inventor: **Phillips, Anthony J.**
Moredown, Standon Road Little Hadham
Ware Hertfordshire SG11 2DD(GB)

(74) Representative: **Hesketh, Alan, Dr.**
European Patent Department Merck & Co., Inc. Terlings Park Eastwick Road
Harlow Essex, CM20 2QR(GB)

(54) **Admixtures of diflunisal and tromethamine.**

(57) An admixture of diflunisal, i.e.,

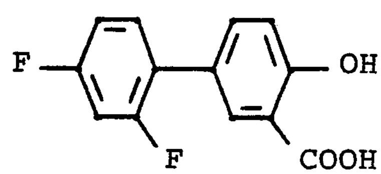

and tromethamine, i.e., $NH_2C(CH_2OH)_3$.

EP 0 230 125 A1

# ADMIXTURES OF DIFLUNISAL AND TROMETHAMINE

## BACKGROUND OF THE INVENTION

Diflunisal, 2',4'-difluoro-4-hydroxy[1,1'-biphenyl]-3-carboxylic acid, has been shown to exhibit analgesic and anti-inflammatory activity in humans. Diflunisal, however, has a slow onset of activity. It was surprisingly found that the compositions of the present invention shorten the onset of activity.

## SUMMARY OF THE INVENTION

This invention relates to admixtures of diflunisal and tromethamine. Diflunisal is disclosed in U.S. Patent 3,714,226, which is hereby incorporated by reference. The admixture of the present invention markedly increased solubility and dissolution rate under aqueous conditions and improved the pharmacokinetic profile when compared with diflunisal alone.

## DETAILED DESCRIPTION OF THE INVENTION

The present invention is directed to an admixture of diflunisal and tromethamine. Diflunisal may be represented by the following structural formula - (A) :

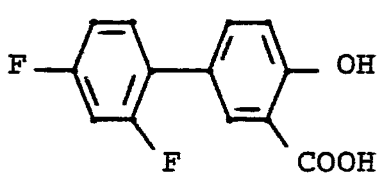

Tromethamine may be represented by the following structure (B) : $NH_2C(CH_2OH)_3$.

The admixture may optionally be used in combination with pharmaceutical carriers.

This invention also relates to a method of treating inflammation in patients using the admixture.

The admixture of the instant invention can be used to treat inflammation by reducing inflammation and relieving pain in such diseases as rheumatoid arthritis, osteoarthritis, gout, infectious arthritis and rheumatic fever. Furthermore, the admixture of the instant invention has better potency at the same dosage levels than similar type compounds known in the prior art and exhibits a lower incidence of side effects.

The admixture also has analgesic activity and would be administered and used in the same manner and in the same dosage ranges as if they were being used to treat inflammation as discussed further on.

The admixture exhibits in addition to potent anti-inflammatory effects a smaller incidence of vomiting (emesis effect) than do similar type compounds of the prior art, especially acetyl salicyclic acid (aspirin) type compounds. The admixture of the instant invention has a better therapeutic ratio than does aspirin.

The treatment of inflammation in accordance with the method of the present invention is accomplished by orally administering to patients a therapeutic dose of the admixture, particularly in a non-toxic pharmaceutically acceptable carrier, preferably in tablet or capsule form.

The non-toxic pharmaceutical carrier may be for example, either as solid or a liquid. Exemplary of solid carriers are lactose, corn starch, gelatin, talc, terotex, stearic acid, magnesium stearate, terra alba, sucrose, agar, pectin, cab-o-sil, and acacia. Exemplary of liquid carriers are peanut oil, olive oil, sesame oil and water.

Several pharmaceutical forms of the therapeutically useful admixture can be used. For example, if a solid carrier is used, the admixture may take the form of tablets, capsules, powders, troches or lozenges, prepared by standard pharmaceutical techniques. If a liquid carrier is used, the preparation may be in the form of a soft gelatin capsule, a syrup or a liquid suspension.

The admixture is used in an amount sufficient to treat inflammation, that is to reduce inflammation. Advantageously, the admixture will contain the active ingredient, namely, diflunisal in an amount of from about 1 mg to 140 mg per kg body weight per day (50 mg to 10 g per patient per day), preferably from about 2 mg to 70 mg per kg body weight per day (100 mg to 5 g per patient per day).

The method of treatment of this invention comprises internally administering to a patient (animal or human), the admixture, particularly admixed with a non-toxic pharmaceutical carrier such as exemplified above. The most rapid and effective anti-inflammatory effect is obtained from oral administration of a daily dosage of from about 4 to 10 mg/kg/day. It should be understood, however, that although preferred dosage ranges are given, the dose level for any particular patient depends upon

the age, body weight, sex, time of administration, route of administration, rate of excretion, drug combination, reaction sensitivities and severity of the particular disease of the patient.

Diflunisal and tromethamine are known compounds and are prepared according to general procedures.

The quantity of tromethamine used may be calculated from:

total No. of mEq = 3.5 + N

where N is the number of milliequivalents of diflunisal in the formulation. The value of 3.5 is the quantity of tromethamine necessary to neutralize the acid contents of the fasting stomach. Thus for a tablet containing 250 mg of diflunisal, the total quantity of tromethamine is 4.5 mEq. The preferred range is a total of 3.5 to 5.5 mEq representing a value for N from 0 to 2.

It is preferred that the admixture comprise 125 to 500 mg of diflunisal and 425 to 905 mg of tromethamine and that the weight ratio of diflunisal to tromethamine be 1.1:1.0 to 1.0:4.4.

The admixture may be prepared by blending diflunisal, tromethamine and a suitable binding agent in a suitable mixer and granulated by addition of water or aqueous-solvent mixtures. The wet mass may be milled, dried, rescreened and blended with disintegrants and lubricants before compression. To prevent the irritancy of diflunisal to the mouth and throat, the tablets may be film coated.

The following Example is used by way of illustration:

## Example

This was an open-label, single-dose, two-way crossover study in 18 healthy subjects. Sixteen completed the following treatments. Treatment days were separated by at least 7 days.

Treatment A: One diflunisal-tromethamine tablet containing 250 mg (1 mEq) diflunisal and 550 mg (4.5 mEq) tromethamine

Treatment B: One DOLOBID (manufactured by Merck & Co. Inc.) tablet containing 250 mg diflunisal

Plasma samples were collected at 0, 0.25, 0.5, 0.75, 1, 1.5, 2, 3, 4, and 6 hours. Complete urine was collected for the following intervals: -1 to 0 hour (control) 0-2, 2-4, 4-6, 6-24, 24-48, 48-72, and 72-96 hours. All samples were kept frozen until the time of analysis.

Table 1 lists the physicochemical properties of the two dosage forms. Table 2 summarizes the pharmacokinetic parameters. Based on the date shown, it is apparent that the total uirnary recovery of diflunisal and the peak plasma level of diflunisal are comparable between the two treatments. However, the time-to-plasma peak appears to be shifted to the left, suggesting an improvement in the absorption rate. The extent of absorption, as judged by the total urinery recoveries, was not affected.

Table 1

Physicochemical Properties of the Test Dosage Forms

| | DOLOBID Tablets | Diflunisal-Tromethamine Tablets |
|---|---|---|
| Composition | | |
| Diflunisal | 250 mg | 250 mg |
| Hydroxypropylcellulose (binding agent) | 8.5 mg | -- |
| LF NF w/0.3% silica | | |
| Starch Pregelatinized NF 1500 (bulking agent/disintegrant) | 104 mg | -- |
| Cellulose Microcrystalline NF (bulking agent/disintegrant) | 43.5 mg | -- |
| Magnesium Stearate NF (lubricant) | 4 mg | 5.25 mg |
| Coating | 10 mg | -- |
| Tromethamine | -- | 550 mg |
| Byco C Gelatin (binding agent) | -- | 40 mg |
| Purified Talc (anti-adherent) | -- | 35 mg |
| Explotab (disintegrant) | -- | 35 mg |
| Coating | | |
| Hydroxypropylmethylcellulose | -- | 5 mg |
| Polyethylene Glycol 400 | -- | 0.3 mg |
| Titanium Dioxide | -- | 0.56 mg |
| Purified Talc | -- | 1.14 mg |
| Total Weight | 420 mg | 922.25 mg |
| Assay Value--for diflunisal | 244 mg | 245.3 mg |
| --for tromethamine | -- | 552 mg |
| Dissolution--Q=80% in 30 minutes | Confirm | -- |
| --in 30 minutes | -- | 101% |
| Dose Uniformity-- | Confirm | -- |
| --Range | -- | 929-946 mg |
| --RSD | -- | 0.45% |

**Table 2**

**Summary of Pharmacokinetic Data[a]**

| | Peak Plasma Level | | | | | Urinary Recovery | | |
|---|---|---|---|---|---|---|---|---|
| | $C_{max=}$mcg/ml | | Ratio | $T_{max=}$hrs | | mg | | Ratio |
| Subj. | D | DT | (DT/D) | D | DT | D | DT | (DT/D) |
| 1 | 32.23 | 35.68 | 1.11 | 2.0 | 2.0 | 124.21 | 114.27 | 0.92 |
| 2 | 27.00 | 39.37 | 1.46 | 1.5 | 1.0 | 150.58 | 127.07 | 0.84 |
| 3 | 25.99 | 33.57 | 1.29 | 1.5 | 1.5 | 161.73 | 160.69 | 0.99 |
| 4 | 35.72 | 32.21 | 0.90 | 3.0 | 1.5 | 114.31 | 105.72 | 0.92 |
| 5 | 31.92 | 22.42 | 0.70 | 1.5 | 0.75 | 170.79 | 140.35 | 0.82 |
| 6 | 26.53 | 49.00 | 1.85 | 4.0 | 1.0 | 148.02 | 146.49 | 0.98 |
| 7 | 31.70 | 31.68 | 1.00 | 3.0 | 2.0 | 170.92 | 127.75 | 0.74 |
| 8 | 27.64 | 44.08 | 1.59 | 4.0 | 2.0 | 91.52 | 167.20 | 1.82 |
| 9 | 31.96 | 37.97 | 1.19 | 1.5 | 1.0 | 191.34 | 154.96 | 0.81 |
| 10 | -- | 36.55 | -- | -- | 0.5 | -- | 113.66 | -- |
| 11 | 41.59 | 37.72 | 0.91 | 3.0 | 2.0 | 132.97 | 116.90 | 0.87 |
| 12 | 32.91 | 25.37 | 0.77 | 1.5 | 2.0 | 165.93 | 156.13 | 0.94 |
| 13 | 29.86 | 45.74 | 1.53 | 3.0 | 0.75 | 118.49 | 125.17 | 1.05 |
| 14 | 30.60 | -- | -- | 2.0 | -- | 142.13 | -- | -- |
| 15 | 37.14 | 50.96 | 1.37 | 1.5 | 0.75 | 93.27 | 119.79 | 1.28 |
| 16 | 37.15 | 38.02 | 1.02 | 1.0 | 1.0 | 165.08 | 171.70 | 1.03 |
| 17 | 24.70 | 27.43 | 1.11 | 4.0 | 3.0 | 165.94 | 140.19 | 0.84 |
| 18 | 30.72 | 33.63 | 1.09 | 6.0 | 0.75 | 148.91 | 160.03 | 1.07 |
| Mean | 31.50 | 36.55 | 1.14* | 2.6 | 1.4 | -- | -- | 0.97** |
| S.D. | ±4.52 | ±7.85 | 0.98 | ±1.3 | ±0.7 | | | 0.87 |
| | | | 1.31 | | | | | 1.09 |

a D = DOLOBID Tablets (Treatment A); DT = diflunidal-tromethamine (treatment B)

* Geometric mean; values in parentheses indicate 95% C.I.

** Corrected for assayed dose.

In the presence of tromethamine, peak plasma levels of diflunisal following oral administration were not--affected; however, peak time was improved. Urinary recovery of diflunisal remained unchanged. This test shows that the time to peak bioavailability of the diflunisal/tromethamine admixture is reduced from 2.6 hours (peak bioavailability of diflunisal alone) to 1.4 hours, so that an earlier clinical onset with the diflunisal/tromethamine admixture is anticipated.

## Claims

1. An admixture, comprising: (A) diflunisal and - (B) tromethamine.

2. The admixture of Claim 1, comprising 125 to 500 mg of diflunisal and 425 to 905 mg of tromethamine.

3. The admixture of Claim 1, wherein the weight ratio of diflunisal to tromethamine is 1.1 to 1.0 to 1.0 to 4.4.

4. A pharmaceutical composition comprising the admixture of Claim 1 and a pharmaceutically acceptable carrier.

5. The used of an admixture of diflunisal and tromethamine for the manufacture of a medicament for the treatment of inflammation and pain.

6. The use of an admixture of diflunisal and tromethamine for the manufacture of a medicament useful for increasing the bioavailability of diflunisal in a patient in need of treatment of inflammation and pain.

<u>Claims for the following contracting states: AT,ES,GR</u>

1. A process for preparing an admixture which process comprises mixing diflunisal and tromethamine.

2. The process of Claim 1, which comprises mixing 125 to 500 mg of diflunisal and 425 to 905 mg of tromethamine.

3. The process of Claim 1, wherein the weight ratio of diflunisal to tromethamine is 1.1 to 1.0 to 1.0 to 4.4.

4. A process for preparing a pharmaceutical composition which process comprises mixing diflunisal and tromethamine with a pharmaceutically acceptable carrier.

European Patent Office

# EUROPEAN SEARCH REPORT

Application number

EP 86 30 9720

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 017 102 (MERCK & CO. INC.)<br>--- | 1-6 | A 61 K 31/615 |
| D,A | US-A-3 714 226 (W.V. RUYLE)<br>----- | 1-6 | |
| | | | TECHNICAL FIELDS SEARCHED (Int. Cl.4) |
| | | | A 61 K |

The present search report has been drawn up for all claims

| Place of search | Date of completion of the search | Examiner |
|---|---|---|
| THE HAGUE | 01-04-1987 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82